Europäisches Patentamt

European Patent Office

Office européen des brevets

(11) Publication number: **0 028 942**
**B1**

# EUROPEAN PATENT SPECIFICATION

(45) Date of publication of patent specification: **18.09.85**

(21) Application number: **80304064.1**

(22) Date of filing: **13.11.80**

(51) Int. Cl.⁴: **C 12 N 11/00, C 12 N 11/14,**
**C 12 N 9/92 // C12R1/045,**
**C12R1/465, C12R1/61,**
**C12R1/49**

(54) Process for the production of an immobilized enzyme system and the purification of glucose isomerase.

(30) Priority: **13.11.79 US 93568**
**13.11.79 US 93570**

(43) Date of publication of application:
**20.05.81 Bulletin 81/20**

(45) Publication of the grant of the patent:
**18.09.85 Bulletin 85/38**

(84) Designated Contracting States:
**AT CH DE FR GB IT LI NL SE**

(56) References cited:
**GB-A-1 371 489**
**US-A-3 622 463**
**US-A-3 753 858**
**US-A-3 834 988**
**US-A-3 992 262**
**US-A-4 003 793**

**Topics in Enzyma and Fermentation**
**Biotechnology, first volume, pp. 147-164, 1977**
**Biochemistry, Sehninger, page 144, 1970**

(73) Proprietor: **UOP Inc.**
**10 UOP Plaza Algonquin & Mt. Prospect Roads**
**Des Plaines Illinois 60016 (US)**

(72) Inventor: **Winans, Vida**
**5625 Dunham**
**Downers Grove, Illinois (US)**
Inventor: **Jackson, Denise Marie**
**3406 Orange Avenue**
**Chicago, Illinois (US)**
Inventor: **Tsuda, Yoshihisa**
**2411 Thornwood**
**Wilmette, Illinois (US)**

(74) Representative: **Hale, Stephen Geoffrey et al**
**J.Y. & G.W. Johnson Furnival House 14/18 High**
**Holborn**
**London WC1V 6DE (GB)**

Courier Press, Leamington Spa, England.

**Description**

This invention relates to the purification of a thermally stable enzyme, the enzyme glucose isomerase, and to the production of an immobilized enzyme system.

It is known that fructose is substantially sweeter than glucose. Because the latter is relatively inexpensive and readily available, it is desirable to have an efficient and economical means of converting glucose to fructose. The alkaline isomerization of glucose yields fructose, but the production of undesirable side products and the necessity of removing caustic and other materials from a food ingredient make this route unattractive. A more preferred isomerization method utilizes enzymes and this has the advantages of specificity of reaction and lesser likelihood of producing undesirable side products which have to be removed before the fructose-containing material can be used in foods. The enzymes which effect the conversion of glucose to fructose are called glucose isomerases and are formed by bacteria such as those belonging to the genus *Arthrobacter* and the genus *Actinoplanes*. These enzymes are water-soluble, and if they are merely added to aqueous solutions of glucose the recovery of the enzyme for reuse is difficult and expensive. Using the enzyme only once also affords a process which is relatively expensive. Consequently, many techniques have been developed for immobilizing the enzyme in such a way that substantial enzymatic activity in isomerizing glucose to fructose is displayed while the enzyme itself remains rigidly attached to some water-insoluble support, thereby permitting reuse of the enzyme over substantial periods of time and for substantial amounts of glucose-containing solutions. One example of a method for immobilizing an enzyme is to be found in U.S. Patent 4,141,857, where a polyamine is absorbed on a metal oxide such as alumina, the resulting composite is treated with an excess of a bifunctional reagent, such as glutaraldehyde, so as to crosslink the amine, thereby entrapping the resulting polymer in the pores of the metal oxide, and thereafter the mass is contacted with an enzyme to form covalent bonds between the pendant aldehyde groups and amino groups on the enzyme. It is highly desirable that the material used in making the immobilized enzyme system should contain the desired enzyme, here glucose isomerase, in as chemically pure a state as possible, both to ensure maximum loading on the support and to ensure that the immobilized enzyme system will be homogeneous in respect of the kind of enzyme bound to the support, thereby ensuring maximum specificity, in the conversion of glucose to fructose in this case.

US—A—3,753,858 teaches a method of fixing glucose isomerase in a microorganism from the streptomyces genus by heat treating the microorganism. By this heat treatment the cell structure is preserved and the glucose isomerase is fixed intercellularly in an insoluble state along with other cell matter. In contradistinction, Applicant has discovered that heat treatment of glucose isomerase containing compositions may be used to separate glucose isomerase from other cell matter thereby producing a purified glucose isomerase.

GB—A—1,371,489 discloses a process in which a slurry of cells of *Streptomyces* SP (ATCC 21175) is contacted with CoCl and a cationic detergent and stirred for 3.75 hours at 60°C and a pH of 6.7 to 6.8 and the resulting filtrate is treated with DEAE-cellulose to immobilise the glucose isomerase separated from the cells on the cellulose. The resulting material is used to isomerise the glucose to fructose. Chapter 7 of "Topics in enzyme and fermentation biotechnology", Volume 1, pages 147—164 (1977) refers to that disclosure.

This invention seeks to provide an alternative method of producing an immobilized glucose isomerase system in which the glucose isomerase has been purified in a simple but effective manner prior to immobilization.

According to one aspect of the invention, a method of purifying glucose isomerase prior to immobilizing it on a solid support to form an immobilized enzyme system comprises heat treating a suspension of cells containing the glucose isomerase, rupturing the cell walls after the heat treatment so as to release their contents, removing the solid matter formed by the heat treatment and recovering a purified product, e.g. a solution, comprising the enzyme. Preferably, the recovered glucose isomerase is immobilized on a support matrix comprising gamma-alumina coated with polyethyleneimine, subsequently cross-linked with an excess of glutaraldehyde so as to furnish a plurality of functional pendant groups. In a still more specific embodiment the glucose isomerase has been produced by a microorganism of the genus *Actinoplanes* or the genus *Streptomyces*.

This invention also seeks to provide a method whereby a glucose isomerase solution may be further purified after such a heat treatment, for example after a solution containing glucose isomerase has been heated in the above temperature range for a period of from 5 to 60 minutes and then been cooled to a temperature of from 0°C to 20°C, and the precipitated material discarded.

According to a second aspect of the invention a process for purifying the enzyme glucose isomerase comprises treating an enzyme solution containing glucose isomerase with an acid in an amount sufficient to result in the solution having a pH greater than 3.5 µ up to 4.5, thereby precipitating proteinaceous solids, and collecting the proteinaceous solids, resuspending these solids in a buffer solution having a pH of from 6 to 8 and collecting the resulting extract solution, and further purifying the enzyme solution by salt fractionation wherein a salt is added to the extract solution in an amount corresponding to 40% to 45% of its saturation amount and the solids which precipitate from this solution are discarded, and thereafter additional salt is dissolved in the salt solution so as to attain from 46% to 60% of its saturation amount and the solids containing the purified enzyme are then collected. A nine-fold increase in specific activity, expressed in units of activity per milligram of protein, with total glucose isomerase recoveries of up to 85%, may be realized by this procedure.

Purification of an enzyme to be immobilized on a support matrix and to be utilized thereafter in an immobilized enzyme system has several purposes. One purpose is to maximize efficiency of immobilization, which is to say that it is desirable to immobilize the maximum amount of enzyme which is presented to a support matrix. Such a goal may be achieved by selectively removing from the enzyme preparation those materials which interfere with the enzyme immobilization process, or which compete with enzyme for sites on the support matrix which are responsible for enzyme immobilization. Another purpose of purification is to eliminate the materials which may be immobilized along with the enzyme and whose presence would be deleterious in some way to the desired enzymatic conversion. Such deleterious action would include a decrease in enzymatic activity, promotion of undesirable concurrent conversions, and a decrease in useful lifetime of the enzyme system.

In this context, purification of an enzyme may be defined in two somewhat different ways. On the one hand, purification may be associated with a selective increase in the proportion of enzyme present relative to the total proteinaceous matter. This definition is akin to the more-or-less conventional chemical concept of purification and is the traditional definition of purification. In the case of enzymes, this concept of purification is measured by specific activity of the enzyme preparation, i.e. units of enzyme activity per mg of proteinaceous matter. On the other hand, purification may be associated with achievement of a condition or status of an enzyme-containing preparation such that the support matrix is "blind" to the presence of components other than enzyme. This definition is akin to selective removal of only those components which may interfere with the intended use of the desired component. In the case of enzymes used in immobilized enzyme systems, this latter concept of purification is measured by operational indicia, e.g. efficiency in immobilizing enzyme offered to the support matrix, specific activity of the immobilized enzyme system, specificity of the desired enzymatic reaction, useful lifetime of the immobilized enzyme system and stability of the immobilized enzyme system. It must be emphasised that in this latter concept of enzyme purification specific activity of the enzyme preparation is an inadequate index and even may be misleading. Unless stated otherwise, the term "purification" when used herein relates to this latter concept of enzyme purification, in contradistinction to the traditional concept of purification.

The prior art describes a process for purification of glucose isomerase as exemplified in U.S. Patent 4,077,842. In this method a water-miscible organic solvent, such as isopropyl alcohol, is added to an aqueous solution of the enzyme, resulting in precipitation of enzyme-inactive matter. Solids are removed, and the solution is treated with a soluble magnesium salt, thereby causing precipitation of an enzyme-magnesium complex which is recovered by suitable means. This method exemplifies traditional purification techniques.

The necessity of resorting to extensive methods of purification under some circumstances is not denied. For example, such a method of purification may be desirable where the enzyme needs to be stored for long periods, especially in a dried state. However, we have found unexpectedly that a much simpler, yet equally effective, expedient, namely thermal treatment, is available where a thermally stable enzyme is to be used in an immobilized enzyme system. In particular, heat treatment of a crude glucose isomerase-containing composition causes precipitation of materials whose removal affords a solution of glucose isomerase from which the enzyme can be efficiently immobilized by a support matrix with the resulting immobilized enzyme system being operationally equivalent to those prepared from enzyme purified by traditional methods, such as the one described in the prior art.

Glucose isomerase is an example of a thermally stable enzyme. Enzymes with glucose isomerase activity are produced by many microorganisms, including those of the genus *Streptomyces, Lactobacillus, Curtobacterium*, and *Actinoplanes*. Examples of particular species of glucose isomerase producers from the above genera include: *A. missouriensis, A. philippinensis, A. armeniacus, L. pentosus, L. breves, C. citreum, C. luteum* and *C. helvolum*, etc. The Streptomyces are particularly rich in glucose isomerase producers, and examples of such species include *S. olivochromogenes, S. venezuelae, S. coelicolor, S. aureus, S. griseolus*, and *S. virginiae*. By way of illustration only, *A. missouriensis* may be cultured on a medium containing a suitable carbon source and other appropriate nutrients for a time sufficient to give maximum, or near maximum, glucose isomerase activity. Whole cells containing the enzymes are collected by suitable means such as filtration, washed, then freeze dried and resuspended in a buffer at a pH of 6 to 8. In a preferred embodiment the buffer is selected from imidazole, phosphate and tris(hydroxymethyl-amino)methane, including mixtures of two or all thereof. It is contemplated within the scope of this invention that other buffers may be used, but not necessarily with equivalent results. In addition, if so desired, the buffered solution may also contain divalent cobalt ions at a concentration in the range from $10^{-4}$ to $10^{-2}$ molar, and magnesium ions at a concentration in the range from $10^{-3}$ to $10^{-1}$ molar.

Such a buffered solution is then subjected according to the invention to a heat treatment which performs the dual function of deactivating other enzymes and precipitating undesired material. Such treatment suitably comprises maintaining the glucose isomerase preparation at a temperature from 40°C to 80°C for a period of from 5 minutes to 120 minutes. In a preferred embodiment the enzyme preparation is heated to from 55°C to 65°C for a period of from 10 to 30 minutes or longer. The preparation then is preferably cooled rapidly to a temperature of from 0°C to 20°C and material which precipitates is removed by suitable means, centrifugation for example, and discarded.

To release the enzyme from the cells the cell walls must be ruptured. Examples of suitable means include chemical rupture, as by digestion with a lysozyme enzyme preparation, or physical rupture, as by rending the walls with sound waves (sonication) or mechanical grinding. In one embodiment the enzyme may be released by sonication at a temperature between 0°C and 15°C. The cell debris which is formed, together with material precipitated by the heat treatment, may be removed by any means known to those skilled in the art, for example by centrifugation, to afford a solution containing the enzyme, glucose isomerase. The clear supernatant solution may then be immobilised directly.

If it is so desired, the resultant enzyme preparation may be concentrated as by ultrafiltration and/or the preparation may be dialyzed against a suitable buffer. Alternatively, the enzyme preparation resulting from heat treatment may be utilized as the crude enzyme source in a more elaborate purification scheme, such as those based on solvent precipitation, or isoelectric point precipitation, or salt precipitation. It is to be emphasised, however, that immobilization of enzyme from the heat-treated preparation alone gives an immobilized enzyme system which is operationally equivalent to that obtained from the more highly purified preparations.

In those instances where an extensive purification of glucose isomerase is desired, a procedure subsequent to the heat treatment of the enzyme involving acid treatment salt fractionation may be used. Following the heat treatment at a temperature of from 40°C to 80°C for a period from 5 to 60 minutes, the mixture may be cooled rapidly to 0°C to 20°C and the precipitated material discarded.

The subsequent operations in the present process are conveniently performed at temperatures in the range from 0°C to 20°C, and preferably in a range of from 0°C to 5°C. The cooled solution is acidified, generally until the pH is in the range from 3.5 to 5.0. In the case of glucose isomerase from *A. missouriensis* the preferred pH range is from 3.6 to 4.4. Examples of acids which may be used for the acidification are inorganic acids such as hydrochloric acid, organic carboxylic acids such as citric, acetic, propionic and butyric acids and organic sulfonic acids such as benzenesulfonic acid, toluene sulfonic acid and methanesulfonic acid. It is to be understood that these acids are not limiting on the invention, and that other acids may be used, but not necessarily with equivalent results. In a preferred embodiment of the invention the acid employed is acetic acid. The solids which form upon acidification may then be collected by suitable means, such as centrifugation, and subsequently resuspended in a buffer, preferably a buffer selected from the aforementioned group of buffers, at a pH from 6 to 8. This buffer may also contain divalent cobalt ions or magnesium ions or a mixture thereof, in their aforementioned molarity ranges. Such resuspension is equivalent to extraction of glucose isomerase because not all of the solids obtained upon acidification are soluble in said buffer, and these solids are removed by suitable means and discarded.

The enzyme in solution may now be further purified by salt fractionation. A salt is added to the cooled solution in an amount typically corresponding to 40% to 50% of its saturation amount (i.e. the total amount of salt which can be dissolved in the solution). In a preferred embodiment, the salt has a solubility such that at least a 4 molar solution in water at 0°C can be obtained, but is otherwise without limitation. Examples of such salts include ammonium sulfate, ammonium acid sulfate, sodium chloride, potassium acetate, potassium carbonate, and potassium chloride. In another preferred embodiment the salt is an alkali metal or alkaline earth metal sulfate, such as $Li_2SO_4$, $Na_2SO_4$, $K_2SO_4$, $Rb_2SO_4$, $Cs_2SO_4$ and $MgSO_4$. After allowing the solids to precipitate, suitably for a period from 5 to 60 minutes, preferably for a period from 10 to 20 minutes, the solids may be removed by suitable means and discarded. At this point an additional amount of the salt is dissolved such that the total amount in solution corresponds to from 41 to 60% of its saturation amount, preferably from 50 to 60% of its saturation amount, thereby causing precipitation of glucose isomerase. After a period suitably of from 5 to 60 minutes, but preferably from 10 to 30 minutes, the precipitate of purified glucose isomerase may be collected by suitable means, such as centrifugation.

Salts can be removed from the purified enzyme by any means, for example by dialysis or gel permeation chromatography. The solid which is collected as described above may be redissolved in a buffer solution containing a buffer, preferably selected from the above group, at a pH from 6 to 8, and which may also contain divalent cobalt and/or magnesium ions in the above ranges. This enzyme solution may be dialyzed overnight against the same solvent used to dissolve the enzyme. The process carried out in this way can provide an enzyme with up to 9-fold purification, by which is meant that the specific activity, expressed in units of activity per milligram of protein, is about 9-fold greater for the final enzyme preparation than it was for the initial enzyme preparation. In addition, total recoveries of glucose isomerase can range up to 85%, by which is meant that the total activity of the final enzyme preparation can be up to 85% of that of the initial enzyme preparation.

A surprising discovery is that solutions of certain compositions enhance the time periods during which glucose isomerase can be stored without losing substantial activity, and that these same solutions impart enhanced resistance to thermal deactivation of said glucose isomerase. These solutions contain glycerol in amounts ranging from 5% to 60% on a volume-volume basis, imidazole as a buffer at a pH of 6 to 8, divalent cobalt ion in a concentration of $10^{-4}$ to $10^{-2}$ molar, and magnesium ion in a concentration of $10^{-3}$ to $10^{-1}$ molar. The ability of the aforementioned solutions to impart these desirable properties to the enzyme will be shown in greater detail in the examples which are appended to the specification.

The following experiments illustrate this invention.

## 0 028 942

Experiments 1—4

These experiments show the pH range over which acidification is effective to purify glucose isomerase from *A. missouriensis* by precipitation. In all examples 30 ml of a heat-treated enzyme solution were used. To each was added a sufficient amount of 1N acetic acid to adjust the pH in increments of 0.5 unit from a pH of 5.0 to 3.5. The resulting precipitate was separated from the supernatant solution and dissolved in a phosphate buffer. Total enzyme activity was measured for both the supernatant and the solution of the formed precipitate with the results shown in Table I.

TABLE I

| Experiment | pH | Total units of activity | |
|---|---|---|---|
| | | Supernatant | Precipitate |
| 1 | 5.0 | 1922 | 0 |
| 2 | 4.5 | 1972 | 0 |
| 3 | 4.0 | 0 | 1366 |
| 4 | 3.5 | 0 | 0 |

These data show that this enzyme is not precipitated at pH 4.5 and above, and that it is completely deactivated at pH 3.5 and below. Glucose isomerase from other microorganisms may have similarly narrow ranges of pH within which enzyme precipitation is effectively induced.

Experiments 5—9

Experiments to determine the optimum salt concentration for fractionation of glucose isomerase from *A. missouriensis* were performed in the following manner. To aliquot portions of equal volume of acid-precipitated enzyme in a buffer there was added ammonium sulfate in concentrations ranging from 35—55% of its saturation amount. Solids were separated from the supernatant solution and dissolved in about 3 ml of phosphate buffer. One ml portions each of the supernatant and the solution from precipitated solids were assayed for glucose isomerase activity. Results are shown in Table II.

TABLE II

| Experiment | Saturation level of $(NH_4)_2SO_4$ | Activity per ml | |
|---|---|---|---|
| | | Supernatant | Precipitate |
| 5 | 35% | 82.9 | 0 |
| 6 | 40% | 86.8 | 0 |
| 7 | 45% | 70.4 | 0 |
| 8 | 50% | 42.7 | 38.9 |
| 9 | 55% | 10.0 | 74.8 |

The data of Table II clearly show that when added at levels up to 45% of its saturation amount ammonium sulfate causes selective precipitation of glucose isomerase-inactive proteins. After these are removed, further addition of ammonium sulfate causes selective precipitation of glucose isomerase-active proteins, thereby effecting a purification of glucose isomerase.

## Claims

1. A process for producing an immobilised glucose isomerase system comprising contacting a solid support with a solution containing glucose isomerase which has been obtained from cells of a microorganism and which has been subjected to a heat treatment, characterised in that the heat treatment is performed on a suspension of whole cells to deactivate enzymes other than glucose isomerase and to render undesired material insoluble, thereby enabling purified glucose isomerase to be recovered, the cells are ruptured after the heat treatment so as to release their contents, and the released glucose isomerase in solution is separated from solid debris and insoluble material resulting from the heat treatment and cell rupture prior to contact with the solid support.

2. A method as claimed in claim 1, characterised in that the heat treatment is conducted at a temperature from 40°C to 80°C for a period of from 5 minutes to 120 minutes.

5

3. A method as claimed in claim 1 or 2, characterised in that the glucose isomerase has been produced by microorganisms of the species *Actinoplanes missouriensis, Actinoplanes philippinensis, Actinoplanes armeniacus, Streptomyces olivochromogenes, Streptomyces venezuelae, Streptomyces coelicolor, Streptomyces aureus, Streptomyces griseolus,* or *Streptomyces virginiae.*

4. A method as claimed in any one of claims 1 to 3, characterised in that the solution containing glucose isomerase which is contacted with the solid support is a solution which has been dialyzed after separation from the solid debris and insoluble material.

5. A method as claimed in any of claims 1 to 4, characterised in that the solid support comprises gamm-alumina contacted with polyethyleneimine and glutaraldehyde.

6. A process for purifying the enzyme glucose isomerase which comprises:

(a) treating an enzyme solution containing glucose isomerase with an acid in an amount sufficient to result in the solution having a pH from greater than 3.5 up to 4.5, thereby precipitating proteinaceous solids, and collecting the proteinaceous solids;

(b) extracting the proteinaceous solids with a buffer solution having a pH from 6 to 8 and collecting the extract solution;

(c) dissolving in the extract solution sufficient salt to attain from 40% to 45% of its saturation amount and collecting the resulting salt solution; and

(d) dissolving additional salt in the salt solution from step (c) in an amount sufficient to attain from 46% to 60% of its saturation amount and collecting solids containing purified enzyme.

7. A process as claimed in claim 6, characterised in that the buffer solution contains a buffer selected from imidazole, phosphate and tris(hydroxymethylamino)methane, and/or the buffer solution contains divalent cobalt ions at a concentration from $10^{-4}$ to $10^{-2}$ molar or magnesium ions at a concentration from $10^{-3}$ to $10^{-1}$ molar.

8. A process as claimed in claim 6 or 7, characterised in that the acid is selected from hydrochloric, citric, acetic, propionic, butyric acid and organic sulfonic acids and/or the salts used in steps (c) and (d) have a solubility in water at 0°C at least equivalent to that of a 4 molar solution.

**Patentansprüche**

1. Verfahren zur Gewinnung eines immobilisierten Glukoseisomerasesystems unter Einschluss der Massnahme, dass man einen festen Träger mit einer aus Mikroorganismenzellen erhältlichen und hitzebehandelten glukoseisomerasehaltigen Lösung in Kontakt bringt, dadurch gekennzeichnet, dass man eine Suspension von ganzen Zellen zur Inaktivierung von Enzymen, die von Glukoseisomerase verschieden sind, sowie zum Unlöslichmachen unerwünschter Stoffe hitzebehandelt, wobei die Gewinnung gereinigter Glukoseisomerase ermöglicht wird, die Zellen nach der Hitzebehandlung zur Freisetzung ihres Inhalts aufbricht und die freigesetzte gelöste Glukoseisomerase von den festen Zelltrümmern und von den infolge Hitzebehandlung und Zellenaufschluss entstandenen unlöslichen Stoffen vor der Kontaktierung mit dem festen Träger abtrennt.

2. Verfahren nach Anspruch 1, dadurch gekennzeichnet, dass man die Hitzebehandlung bei einer Temperatur von 40 bis 80°C innerhalb von 5 bis 120 Minuten durchführt.

3. Verfahren nach Anspruch 1 oder 2, dadurch gekennzeichnet, dass die Glukoseisomerase aus den Mikroorganismen der Arten *Actinoplanes missouriensis, Actinoplanes philippinensis, Actinoplanes armeniacus, Streptomyces olivochromogenes, Streptomyces venezuelae, Streptomyces coelicolor, Streptomyces aureus, Streptomyces griseolus* oder *Streptomyces virginiae* gewonnen wurde.

4. Verfahren nach einem der Ansprüche 1 bis 3, dadurch gekennzeichnet, dass die mit dem festen Träger in Berührung gebrachte glukoseisomerasehaltige Lösung eine Lösung darstellt, welche nach Abtrennung von den festen Zelltrümmern und unlöslichen Stoffen dialysiert wurde.

5. Verfahren nach einem der Ansprüche 1 bis 4, dadurch gekennzeichnet, dass der feste Träger mit Polyäthylenimin und Glutaraldehyd in Berührung gebrachtes γ-Aluminiumoxyd enthält.

6. Verfahren zur Reinigung des Enzymes Glukoseisomerase, gekennzeichnet durch die folgenden Schritte:

a) Behandeln einer glukoseisomerasehaltigen Enzymlösung mit einer zur Erzielung eines pH-Werts in der Lösung von mehr als 3,5 bis 4,5 ausreichenden Menge einer Säure, wobei die eiweissartigen Feststoffe ausgefällt und dieselben gesammelt werden;

b) Extrahieren der eiweissartigen Feststoffe, mittels einer einen pH-Wert von 6 bis 8 aufweisenden Pufferlösung und Gewinnung der Extraktlösung;

c) Auflösen einer für eine 40 bis 45%ige Sättigung ausreichenden Menge an Salz in der Extraktlösung und Gewinnung der erhaltenen Salzlösung und

d) Auflösen eines zusätzlichen Quantums an Salz in der Salzlösung aus Schritt c) in einer Menge, die zur Erzeilung einer 46 bis 60%igen Sättigung ausreicht sowie Sammeln der das gereinigte Enzym enthaltenden Feststoffe.

7. Verfahren nach Anspruch 6, dadurch gekennzeichnet, dass die Pufferlösung eine unter Imidazol, Phosphat und Tris(hydroxymethylamino)methan ausgewählte Puffersubstanz und/oder zweiwertige Kobaltionen in einer molaren Konzentration von $10^{-4}$ bis $10^{-2}$ oder Magnesiumionen in einer molaren Konzentration von $10^{-3}$ bis $10^{-1}$ enthält.

8. Verfahren nach Anspruch 6 oder 7, dadurch gekennzeichnet, dass die Säure unter Salz-, Zitronen-, Essig-, Propion-, Buttersäure oder organischen Sulfonsäuren ausgewählt ist, und/oder dass die gemäss Schritt (c) und (d) verwendeten Salze in Wasser bei 0°C eine Löslichkeit besitzen, die wenigstens jener einer 4-molaren Lösung äquivalent ist.

## Revendications

1. Procédé de production d'un système de glucose isomérase immobilisée comportant la mise en contact d'un support solide avec une solution contenant la glucose isomérase qui a été obtenue à partir de cellules d'un microorganisme et qui a été soumise à un traitement thermique, caractérisé en ce que l'on opère le traitement thermique sur une suspension de cellules entières pour désactiver les enzymes autres que la glucose isomérase et pour insolubiliser les matières indésirables, permettant, de ce fait, la récupération de la glucose isomérase purifiée, en ce que les cellules sont brisées après le traitement thermique de façon à libérer leur contenu, et en ce que l'on sépare la glucose isomérase en solution des débris solides et matières insolubles résultant du traitement thermique et de la rupture des cellules, avant la mise en contact avec le support solide.

2. Procédé selon la revendication 1, caractérisé en ce que l'on effectue le traitement thermique à une temperature de 40°C à 80°C, sur une période de 5 minutes à 120 minutes.

3. Procédé selon la revendication 1 ou 2, caractérisé en ce que la glucose isomérase a été produite par des microorganismes des espèces *Actinoplanes missouriensis*, *Actinoplanes philippinensis*, *Actinoplanes armeniacus*, *Streptomyces olivochromogenes*, *Streptomyces venezuelae*, *Streptomyces coelicolor*, *Streptomyces aureus*, *Streptomyces griseolus*, ou *Streptomyces virginiae*.

4. Procédé selon l'une quelconque des revendications 1 à 3, caractérisé en ce que la solution, contenant la glucose isomerase, que l'on met en contact avec le support solide, est une solution qui a été dialysée après séparation des débris solides et des matières insolubles.

5. Procédé selon l'une quelconque des revendications 1 à 4, caractérisé en ce que le support solide contient de l'alumine gamma mise en contact avec de la polyéthylèneimine et de l'aldéhyde glutarique.

6. Procédé de purification de l'enzyme glucose isomérase qui comporte:

(a) le traitement d'une solution enzymatique contenant la glucose isomérase per un acide en quantité suffisante pour que la solution ait un pH de plus de 3,5 jusqu'à 4,5, en précipitant, de ce fait, les solides protéiniques, et la collection des solides protéiniques;

(b) l'extraction des solides protéiniques à l'aide d'une solution tampon dont le pH est entre 6 et 8 et la collection de la solution d'extrait;

(c) la dissolution dans la solution d'extrait d'assez de sel pour atteindre 40% à 45% de sa quantité de saturation et la collection de la solution saline résultante; et

(d) la dissolution de sel supplémentaire dans la solution saline provenant de l'étape (c) en quantité suffisante pour attendre 46% à 60% de sa quantité de saturation et la collection des solides contenant l'enzyme purifiée.

7. Procédé selon la revendication 6, caractérisé en ce que la solution tampon contient un tampon choisi parmi l'imidazole, le phosphate et le tris(hydroxyméthylamino)méthane, et/ou la solution tampon contient des ions cobalt divalents à une concentration molaire de $10^{-4}$ à $10^{-2}$ ou des ions magnésium à une concentration molaire de $10^{-3}$ à $10^{-1}$.

8. Procédé selon la revendication 6 ou 7, caractérisé en ce que l'acide est choisi parmi les acides chlorhydrique, citrique, acétique, propionique, l'acide butyrique et les acides sulfoniques organiques et/ou en ce que les sels utilisés aux étapes (c) et (d) ont une solubilité dans l'eau à 0°C au moins équivalente à celle d'une solution 4 molaire.